Europäisches Patentamt

European Patent Office

Office européen des brevets

(11) Publication number: **0 196 845 B1**

# EUROPEAN PATENT SPECIFICATION

(45) Date of publication of patent specification: **03.11.93**   (51) Int. Cl.5: **G01N  33/531**, G01N 33/574

(21) Application number: **86302130.9**

(22) Date of filing: **21.03.86**

(54) **Processes for the stabilization of prostate specific antigen in natural matrices.**

(30) Priority: **29.03.85 US 717345**

(43) Date of publication of application:
**08.10.86 Bulletin  86/41**

(45) Publication of the grant of the patent:
**03.11.93 Bulletin  93/44**

(84) Designated Contracting States:
**AT BE CH DE FR GB IT LI LU NL SE**

(56) References cited:
**US-A- 4 446 122**

**CHEMICAL ABSTRACTS, vol. 101, no. 3, 16th July 1984, page 304, columns 1, 2, abstract no. 19951m, Columbus, Ohio, US; N. MORISHITA et al.: "Purification and enzyme immunoassay of tumor markers for prostate cancer: prostatic acid phosphatase, prostate-specific antigen and creatine kinase BB", & NIPPON HINYOKIKA GAKKAI ZASSHI 1984, 75(3), 404-413**

**BIOLOGICAL ABSTRACTS, vol. 78, 1984, Philadelphia, US; R.J. LIEDTKE et al.: "Measurement of protate specific antigen by radio immunoassay", & CLIN. CHEM. 1984, 30(5) 649-652**

(73) Proprietor: **HYBRITECH INCORPORATED**
**11095 Torreyana Road**
**San Diego California 92126(US)**

(72) Inventor: **Berger, Tina Susan**
**14211 Bernabe Court**
**San Diego, CA 92129(US)**
Inventor: **Ivor, Linda Patricia**
**814 Moana Drive**
**San Diego, CA 92106(US)**

(74) Representative: **Goldin, Douglas Michael et al**
**J.A. KEMP & CO.**
**14, South Square**
**Gray's Inn**
**London WC1R 5EU (GB)**

EP 0 196 845 B1

ANNALS NEW YORK ACADEMY OF SCI-
ENCES, vol. 417, 1983, pages 383-389, New
York, US; T.M. CHU et al.: "Circulating anti-
body to prostate antigen in patients with
prostatic cancer"

BIOLOGICAL ABSTRACTS, vol. 71, 1981,
Philadelphia, US; T.M. CHU et al.:
"Ouantitation of prostate specific antigen in
serum by a sensitive enzyme immunoas-
say", & CANCER RES. 1980, 40(12), 4658-4662

:L.Hudson, F.C. Hay. Practical Immunology,
Black well, Scientific Publications, Oxford,
London (1989) p. 19120; Römpps Chemie
Lexikon, 8. Aufl., p. 642

## Description

This invention relates to the detection and quantitation of antigenic substances in fluids such as serum. More Particularly, it relates to processes for preparing stable natural matrices for use in immunoassays for prostate specific antigen.

Prostate specific antigen (PSA), a well characterized tumor associated antigen, is a significant diagnostic and prognostic marker in human prostatic carcinoma. As prostate tumor cells release PSA into the bloodstream, PSA concentrations in serum and other body fluids correlate with the progression of primary or metastatic carcinoma. Accordingly, the quantitation of PSA in Patient specimens provides clinicians with an effective means of monitoring a therapeutic regimen and evaluating remission or progression of the disease state.

Present immunoassays for the measurement of PSA in patient fluid samples, such as the immunoradiometric assay (IRMA) and the enzyme-linked immunosorbent assay (ELISA), rely on the use of artificial or synthetic matrices, such as bovine serum albumin, as calibrator matrices and as diluents. As used herein, the term "calibrator matrix" refers to a matrix in which Predetermined concentrations of an antigenic substance may be maintained for the calibration of unknown concentrations of the antigenic substance in patient samples. The term "diluent," as used herein, refers to a matrix for dilution of patient samples having concentrations of an antigenic substance which exceed the range of the immunoassay, permitting measurement of the antigenic substance within the immunoassay range.

These matrices are used because unmodified natural matrices, such as serum-based matrices, are rendered unsuitable for use as a result of the instability of PSA upon introduction into such matrices. Specifically, it has been shown that a 30-70% loss of PSA activity occurs within 24 hours after introduction into unmodified human serum-based matrices. Further, this resultant loss of measurable PSA is not limited to human serum since PSA is also unstable upon introduction into bovine and equine serum-based matrices as well.

Because of the dissimilarity of components of such matrices to specimen components, the kinetic patterns and non-specific binding characteristics of artificial matrices may deviate significantly from serum or other body fluids containing, or suspected of containing PSA. As a result, use of these matrices is inherently a substantial limitation to immunoassays for PSA.

Accordingly, to maximize the accuracy and sensitivity of immunoassays for PSA, it is essential that matrices for calibration and sample dilution be as nearly like patient specimens, particularly with respect to non-specific binding characteristics, as possible.

Accordingly, there exists a need for means by which PSA may be stabilized in natural matrices, such as serum-based matrices, for use in the quantitative determination of PSA in patient specimens.

The present invention provides processes for the stabilization of Prostate specific antigen (PSA) in natural matrices. In that regard, we have unexpectedly found that natural matrices having kinetic patterns and non-specific binding characteristics similar to those of patient specimens can be modified so that PSA is stable therein without substantially altering their desirable properties as matrices.

According to the present invention, therefore, biologic fluid being blood, serum, plasma, cerebral spinal fluid or urine, obtained from a suitable mammal and having kinetic patterns and non-specific binding characteristics the same or substantially the same as the patient sample is modified to inhibit the activity of components of the fluid destabilizing to PSA. The biological fluid is modified by an alkaline pH shift from normal ( about pH 7) to at least pH 9 for a period of time effective to inhibit the activity of fluid components destabilizing to PSA. Thereafter, the pH of the biological fluid is decreased to about pH 7.

This invention has been summarized in order that the detailed description that follows may be better understood, and in order that the contribution to the art may be better appreciated.

As indicated above, the present invention provides a means by which natural matrices may be modified to stabilize prostate specific antigen (PSA). In the context of the present invention, the term "natural matrix" refers to a biologically occurring composition produced by living processes having kinetic patterns and non-specific binding characteristics the same as, or similar enough to, a patient sample to permit its use as a matrix. The present invention is useful for the detection and/or quantitative measurement of PSA in patient fluid samples by means of conventional immunoassay procedures. More specifically, stable natural matrices prepared in accordance with the processes of the present invention are useful as calibrator matrices and diluents in immunoassays for PSA.

Immunoassays for the determination of concentrations of antigenic substances in fluid samples are well known to the art and need not be described in detail. However, among the immunoassays for which the present invention is particularly useful may be mentioned monoclonal antibody-based immunometric assays, such as the "two-site" or "sandwich" immunometric assays described in U.S. Patent No. 4,376,110.

3

Additionally, among the means by which such immunoassays may be accomplished may be mentioned radiometric means, enzymatic means and fluorometric means.

In accordance with the present invention, a biological fluid obtained from a suitable mammal is utilized. Whole blood, serum, plasma, cerebral spinal fluid or urine may be suitably utilized in the present invention. Preferred for use is serum derived from human blood in which the presence of circulating PSA, if any, is not detectable in significant concentrations. Particularly preferred for use is serum derived from human female blood due to the absence of circulating PSA.

According to the presently preferred process of the present invention, human female serum is modified to inhibit the activity of serum components which destabilize PSA. While Applicant does not intend to be bound by any theory for the instability of PSA in unmodified serum, it is believed that such instability is attributable at least in part to the presence of circulating prostate antigen binding protein (PABG). PABG, while present in significantly elevated levels in the serum of males diagnosed as having prostatic carcinoma, has also been shown to be present in normal male and female serum. Chu et al., Annals NYAS, Vol. 417, pp. 383-389, 1983.

Modification of human female serum to provide a stable serum-based matrix for PSA is accomplished by an alkaline pH shift to a pH of at least pH 9 for a period of time effective to inhibit the activity of serum components destabilizing to PSA. Preferably, the serum pH is shifted to about pH 12, the serum is incubated for about 5 to about 45 minutes and thereafter the serum pH is decreased to about pH 7. Modification of the serum to effect pH changes in accordance with the present invention may be accomplished by conventional procedures well known to the art.

The advantages of stable natural matrices prepared in accordance with the present invention when compared with synthetic matrices are apparent by reference to Table I. As the non-specific binding characteristics of such stable natural matrices are comparable to patient specimens, the sensitivity and accuracy of an immunoassay for PSA is enhanced.

Additionally, the stability of PSA in natural matrices modified in accordance with the present invention renders such matrices highly effective and desirable as calibrator matrices and specimen diluents in immunoassays for PSA. As indicated by Tables II and III, PSA is substantially more stable in such matrices as compared with unmodified natural matrices.

Furthermore, it will be appreciated by those skilled in the art having the benefit of this disclosure that the present invention suggests processes for the stabilization of other antigens which are unstable upon introduction into unmodified natural matrices. For example, we have obtained similar results with the tumor-associated antigen calcitonin.

The present invention may be better understood by reference to the following non-limiting example.

EXAMPLE 1

Preparation of pH Modified Serum

Human female serum, obtained from the Interstate Blood Bank, Memphis, Tennessee and maintained at -20°C, was used to prepare a pH modified natural matrix. 1000 ml of the serum was adjusted to approximately pH 12 by addition of 17-20 ml of 10N NaOH in a vessel fitted with a stirring device. The basic serum solution was thereafter incubated for 30 minutes at 22-28° C with stirring, followed by a return to approximately pH 7.0 by the addition of 15-17 ml of 10N HCl. The pH 7 solution was thereafter centrifuged for 10 minutes at 1,000 x g and filtered through a final sieve of 0.2 $\mu$m.

Alternate Method for Preparation of pH Modified Serum

Approximately 0.30 ml to 0.50 ml of 10N NaOH was added with stirring to 20 ml of pooled human female serum to increase the pH to 12.0. The basic serum was incubated for 30 minutes followed by addition of 0.1M Sodium Phosphate (0.276 g monobasic sodium phosphate) with stirring to yield a slight decrease in pH. Thereafter 0.40 ml to 0.60 ml of 6N HCl was added to adjust the pH to pH 6 and the solution was stirred at room temperature for 2-3 hours. The pH was finally adjusted to pH 7 using approximately 0.10 ml to 0.5 ml IN NaOH.

Comparison of Non-Specific Binding

Non-specific binding values attributable to synthetic matrix samples comprising 5% Bovine Serum Albumin (Miles Laboratories, Inc., Elkhart, Indiana)/2% IgG (Pel-Freeze Biologicals, Rogers, Arkansas) were compared with unmodified human female serum samples and pH modified serum samples prepared as described above non-specific binding values were determined using a commercially available two-site immunoradiometric assay, TANDEM®-RPSA (Hybritech Incorporated, San Diego, California). Each matrix was evaluated in a 12 replicate assay using 0.05 ml per tube in accordance with the TANDEM®-R PSA protocol with an extended incubation period of 4 hours.

Table I hereinafter sets forth the average value obtained for the synthetic matrix samples, unmodified serum samples and the pH modified serum samples, respectively. The data is expressed as counts/minute and concentration of PSA (ng/ml) relative to a standard curve.

Table I

| Sample Matrix | CPM | PSA (ng/ml) |
|---|---|---|
| 5% BSA/2% IgG | 1179 | 0.24 |
| Unmodified Serum | 930 | 0.07 |
| pH Modified Serum | 946 | 0.09 |

From Table I it is shown that the non-specific binding values obtained for the unmodified and pH modified serum samples were comparable, while the non-specific binding values obtained for the synthetic samples were significantly higher.

Determination of the Stability of PSA

An accelerated stability study of PSA was performed using unmodified human female serum and pH modified serum prepared as described above. A 10 $\mu$l quantity of 855 ng/ml PSA from seminal plasma was diluted into 990 $\mu$l of each matrix sample and maintained at 35°C. The stability of PSA, set forth in Table II as percent loss of measurable PSA, was determined for each sample at 1, 3 and 5 days relative to a -70°C control sample. By procedures well known to the art, the values obtained may be extrapolated to 1.2, 3.6 and 6.0 months, respectively at 4°C.

Table II

| Sample Matrix | % Loss in Measurable PSA Days at 35°C | | |
|---|---|---|---|
| | 1 | 3 | 5 |
| Unmodified | 21 | 32 | 36 |
| pH 12 modified | 4 | 3 | 0 |
| Unmodified | 20 | 26 | 30 |
| pH 12 modified | 8 | 7 | 5 |

From Table II it is readily apparent that PSA was substantially more stable in the pH modified serum as compared with the unmodified serum.

Determination of the Recovery of PSA

The loss of measurable PSA from either seminal plasma or human male serum following dilution into untreated serum or serum modified as described above was determined. Into 4 tubes containing 0.4 ml of sample matrix, 0.1 ml of 125 ng/ml PSA from seminal plasma was added to yield an expected concentration of 25 ng/ml PSA. An aliquot of each solution was then diluted 1:1 into human male serum previously analyzed to contain 19.1 ng/ml PSA. Each of the four tubes were evaluated for PSA content using a commercially available two-site immunoradiometric assay, TANDEM®-R PSA, (Hybritech Incorporated, San Diego, California), with an extended incubation period of 4 hours. The resulting concentrations and calculated percent loss of PSA are shown in Table III. The data clearly indicates a highly desirable recovery of PSA for the pH modified matrix, while significant loss of PSA occurs in unmodified serum.

Table III

Addition of PSA From Seminal Plasma

|  | PSA Expected ng/ml | PSA Observed ng/ml | PSA % Loss |
|---|---|---|---|
| Unmodified Serum | 25.0 | 12.33 | 51 |
| pH Modified Serum | 25.0 | 24.16 | 3 |

Dilutions of Solutions 1:1 with Human
Serum Containing PSA

|  | PSA Expected ng/ml | PSA Observed ng/ml | PSA % Loss |
|---|---|---|---|
| Unmodified Serum | 15.72 | 15.29 | 3 |
| pH Modified Serum | 21.63 | 22.17 | 0 |

**Claims**

1. A process for preparing a stable natural matrix suitable for use in the measurement of prostate specific antigen (PSA) in a fluid sample by means of an immunoassay comprising modifying a mammalian biological carrier fluid for the above antigen, said fluid being blood, serum, plasma, cerebral spinal fluid or urine, by increasing the pH of the carrier fluid to at least pH 9 for a period of time effective to deactivate components destabilizing to PSA present in said biological carrier fluid that can interfere with the measurement of PSA in the fluid sample, and decreasing said pH to about pH7.

2. The process according to claim 1 wherein said biological carrier fluid comprises blood, serum or plasma.

3. The process according to claim 1 or 2 wherein said mammal is a human.

4. The process according to claim 3 wherein said mammal is a female.

5. The process according to claim 1 wherein the pH of said biological fluid is increased to about pH 12.

6. A process for stabilizing prostate specific antigen (PSA) in a fluid sample suitable for use in the measurement of said PSA comprising;
   (a) obtaining human female serum;
   (b) increasing the pH of said serum to at least pH 9 effective to deactivate any PSA or components destabilizing to PSA present in said serum that can interfere with the measurement of PSA;
   (c) thereafter decreasing the pH of said serum to about pH 7 to form a stable natural matrix; and
   (d) contacting said stable natural matrix to said PSA.

7. The process according to claim 6 wherein said stable natural matrix is used as a calibrator matrix.

8. The process according to claim 6 wherein said stable natural matrix composition is used as a diluent.

9. The process according to claim 6, 7 or 8 wherein the pH of said serum is increased to about pH 12.

10. A stable natural matrix producible by the process of any of Claims 1 to 5.

11. Use of a natural matrix obtainable by the process of any one of claims 1 to 5 as a calibrator matrix.

**12.** Use of a natural matrix obtainable by the process of any one of claims 1 to 5 as a diluent.

**13.** Use of a matrix obtainable by the process of any one of claims 1 to 5 in an immunoassay.

**14.** Use according to claim 13 wherein said immunoassay is a monoclonal antibody-based immunometric assay.

**15.** Use according to claim 14 wherein said immunoassay is a "two-site" immunometric assay.

**16.** Use according to claim 14 wherein the said immunometric assay is accomplished by means selected from the group consisting of radiometric means, enzymatic means and fluorometric means.

**Patentansprüche**

**1.** Verfahren zur Herstellung einer stabilen natürlichen Matrix, die geeignet ist zur Messung des Prostatspezifischen Antigens (PSA) in einer Fluidprobe mittels eines Immunoassays, umfassend die Modifizierung eines biologischen Säugetier-Trägerfluids für das vorstehende Antigen, wobei dieses Fluid Blut, Serum, Plasma, Cerebral-spinales Fluid oder Urin ist, durch Erhöhung des pH-Werts des Trägerfluids auf mindestens pH = 9 während eines Zeitraums, der wirksam ist, um die in diesem biologischen Trägerfluid vorhandenen PSA destabilisierenden Komponenten, die die Messung des PSA in der Fluidprobe stören können, zu deaktivieren, und Verringerung des pH-Werts auf ca. pH = 7.

**2.** Verfahren nach Anspruch 1, dadurch gekennzeichnet, daß das biologische Trägerfluid Blut, Serum oder Plasma umfaßt.

**3.** Verfahren nach Anspruch 1 oder 2, dadurch gekennzeichnet, daß das Säugetier ein Mensch ist.

**4.** Verfahren nach Anspruch 3, dadurch gekennzeichnet, daß das Säugetier eine Frau ist.

**5.** Verfahren nach Anspruch 1, dadurch gekennzeichnet, daß der pH-Wert des biologischen Fluids auf ca. pH = 12 erhöht wird.

**6.** Verfahren zur Stabilisierung von Prostatspezifischem Antigen (PSA) in einer Fluidprobe, die zur Messung dieses PSA geeignet ist, umfassend:
(a) Gewinnung eines menschlichen weiblichen Serums;
(b) Erhöhen des pH-Werts dieses Serums auf mindestens pH = 9, der wirksam ist, um PSA oder Komponenten, die gegenüber dem in diesem Serum vorhandenen PSA destabilisierend sind, und die die Messung des PSA stören können, zu deaktivieren;
(c) nachfolgende Senkung des pH-Werts dieses Serums auf einen pH-Wert von ca. 7, um eine stabile natürliche Matrix zu bilden; und
(d) in-Kontakt-bringen dieser stabilen natürlichen Matrix mit dem PSA.

**7.** Verfahren nach Anspruch 6, dadurch gekennzeichnet, daß die stabile natürliche Matrix als Kalibratormatrix verwendet wird.

**8.** Verfahren nach Anspruch 6, dadurch gekennzeichnet, daß die stabile natürliche Matrixzusammensetzung als Diluens verwendet wird.

**9.** Verfahren nach Anspruch 6, 7 oder 8, dadurch gekennzeichnet, daß der pH-Wert des Serums auf ca. pH = 12 erhöht wird.

**10.** Stabile natürliche Matrix, erhältlich durch ein Verfahren nach einem der Ansprüche 1 bis 5.

**11.** Verwendung einer durch das Verfahren nach einem der Ansprüche 1 bis 5 erhältlichen natürlichen Matrix als Kalibratormatrix.

**12.** Verwendung einer durch das Verfahren nach einem der Ansprüche 1 bis 5 erhältlichen natürlichen Matrix als Diluens.

**13.** Verwendung einer nach dem Verfahren nach einem der Ansprüche 1 bis 5 erhältlichen Matrix in einem Immunoassay.

**14.** Verwendung nach Anspruch 13, dadurch gekennzeichnet, daß der Immunoassay ein immunometrischer Assay auf der Basis monoklonaler Antikörper ist.

**15.** Verwendung nach Anspruch 14, dadurch gekennzeichnet, daß der Immunoassay ein "2-Stellen"-immunometrischer Assay ist.

**16.** Verwendung nach Anspruch 14, dadurch gekennzeichnet, daß der immunometrische Assay ausgeführt wird durch Mittel ausgewählt aus der Gruppe bestehend aus radiometrischen Mitteln, enzymatrischen Mitteln und fluorometrischen Mitteln.

**Revendications**

**1.** Procédé pour l'obtention d'une matrice naturelle stable appropriée pour le dosage de l'antigène spécifique de la prostate (PSA) dans un fluide échantillon à l'aide d'un essai immunologique comprenant la modification d'un fluide biologique de mammifère transporteur de l'antigène ci-dessus, ledit fluide étant du sang, du sérum, du plasma, du liquide céphalo-rachidien ou de l'urine, par augmentation du pH du fluide transporteur au moins à pH 9 pendant une durée efficace pour désactiver les composants déstabilisant le PSA présents dans ledit fluide transporteur qui peuvent interférer avec le dosage du PSA dans le fluide échantillon, et en abaissant ledit pH à un pH voisin de 7.

**2.** Procédé selon la revendication 1, dans lequel le fluide biologique transporteur comprend du sang, du sérum ou du plasma.

**3.** Procédé selon la revendication 1 ou 2, dans lequel ledit mammifère est un humain.

**4.** Procédé selon le revendication 3, dans lequel ledit mammifère est une femme.

**5.** Procédé selon la revendication 1, dans lequel le pH dudit fluide biologique est augmenté à une valeur voisine de pH 12.

**6.** Procédé de stabilisation de l'antigène spécifique de la prostate (PSA) dans un fluide échantillon approprié pour le dosage dudit PSA comprenant :
(a) l'obtention de sérum de femme ;
(b) l'augmentation du pH dudit sérum au moins à pH 9, de façon à désactiver tout PSA ou composants déstabilisants du PSA présents dans ledit sérum qui peuvent interférer avec les mesures du PSA
(c) puis l'abaissement du pH dudit sérum à un pH voisin de 7 pour former une matrice naturelle stable et
(d) la mise en contact de ladite matrice naturelle stable et dudit PSA.

**7.** Procédé selon la revendication 6, dans lequel on utilise ladite matrice naturelle stable en tant que matrice de calibrage.

**8.** Procédé selon la revendication 6, dans lequel on utilise la composition de ladite matrice naturelle stable en tant que diluant.

**9.** Procédé selon la revendication 6,7 ou 8, dans lequel on augmente le pH dudit sérum à un pH voisin de 12.

**10.** Matrice naturelle stable pouvant être obtenue à l'aide du procédé selon l'une quelconque des revendications 1 à 5.

**11.** Utilisation d'une matrice naturelle pouvant être obtenue par le procédé selon l'une quelconque des revendications 1 à 5, en tant que matrice de calibrage.

8

**12.** Utilisation d'une matrice naturelle pouvant être obtenue par le procédé selon l'une quelconque des revendications 1 à 5, en tant que diluant.

**13.** Utilisation d'une matrice pouvant être obtenue par le procédé selon l'une quelconque des revendications 1 à 5, dans un essai immunologique.

**14.** Utilisation selon la revendication 13, dans laquelle ledit essai immunologique est un dosage immunométrique à base d'anticorps monoclonal.

**15.** Utilisation selon la revendication 14, dans laquelle ledit essai immunologique est un essai immunométrique à "deux sites".

**16.** Utilisation selon la revendication 14, dans laquelle ledit essai immunométrique est réalisé à l'aide de moyens choisis dans le groupe constitué par des moyens radiométriques, des moyens enzymatiques et des moyens fluorométriques.